(19) Europäisches Patentamt

European Patent Office

Office européen des brevets

(11) **EP 1 183 244 B1**

(12) **EUROPÄISCHE PATENTSCHRIFT**

(45) Veröffentlichungstag und Bekanntmachung des Hinweises auf die Patenterteilung:
**28.04.2004 Patentblatt 2004/18**

(51) Int Cl.[7]: **C07D 251/28**

(86) Internationale Anmeldenummer:
**PCT/EP2000/002013**

(21) Anmeldenummer: **00912567.5**

(22) Anmeldetag: **08.03.2000**

(87) Internationale Veröffentlichungsnummer:
**WO 2000/064879 (02.11.2000 Gazette 2000/44)**

(54) **VERFAHREN ZUR HERSTELLUNG VON CYANURCHLORID**

METHOD OF PRODUCING CYANURIC CHLORIDE

PROCEDE POUR PRODUIRE DU CHLORURE CYANURIQUE

(84) Benannte Vertragsstaaten:
**BE CH DE LI**

(30) Priorität: **22.04.1999 DE 19918245**

(43) Veröffentlichungstag der Anmeldung:
**06.03.2002 Patentblatt 2002/10**

(73) Patentinhaber: **Degussa AG**
**40474 Düsseldorf (DE)**

(72) Erfinder:
• **BÖRNER, Walter**
**D-63579 Freigericht (DE)**
• **MARQUARDT, Ralph**
**D-60318 Frankfurt am Main (DE)**
• **SCHAUHOFF, Stephanie**
**D-60385 Frankfurt am Main (DE)**
• **SCHICK, Christine**
**D-63067 Offenbach (DE)**
• **VANHEERTUM, Rudolf**
**B-2930 Brasschaat (BE)**

(56) Entgegenhaltungen:
**US-A- 3 312 697       US-A- 3 707 544**
**US-A- 3 867 382**

## Beschreibung

Die Erfindung betrifft ein Verfahren zur Herstellung von Cyanurchlorid durch Trimerisierung von Chlorcyan bei einer Temperatur oberhalb 200 °C an einem Aktivkohlekatalysator. Das erfindungsgemäße Verfahren führt zu einem verminderten spezifischen Katalysatorverbrauch.

Cyanurchlorid wird großtechnisch hergestellt durch Chlorierung von Cyanwasserstoff unter Bildung von Chlorcyan und Trimerisierung des Chlorcyans zu Cyanurchlorid - siehe Ullmann's Encyclopedia of Industrial Chemistry Vol. A8, 5th ed. (1987), 196-197. Die Trimerisierung erfolgt in der Gasphase bei einer Temperatur oberhalb 200 °C, insbesondere im Bereich von etwa 300 bis 450 °C, an einem Aktivkohlekatalysator. Aufgrund der Exothermie der Trimerisierungsreaktion bildet sich im kontinuierlichen Betrieb ein Temperaturprofil entlang der Längsachse des Reaktors; es kommt zur Ausbildung eines sogenannten hot-spot, dessen Temperaturmaximum vom Durchsatz abhängt und mit wachsendem Durchsatz ansteigt. Es ist bekannt, daß der Aktivkohlekatalysator in Abhängigkeit von den Betriebsbedingungen, dem Durchsatz und der Aktivkohlequalität desaktiviert. Die Desaktivierung macht sich dadurch bemerkbar, daß die Reaktionszone und damit das Temperaturmaximum entlang der Längsachse des Katalysators wandert.

Aufgrund der Desaktivierung des Katalysators muß dieser periodisch ausgetauscht oder anderweitig aktiviert werden. Die Wirtschaftlichkeit des Cyanurchloridverfahrens hängt maßgeblich von der Standzeit des Katalysators ab, da nicht nur die Katalysatorkosten, sondern auch die Anlagenstillstandskosten berücksichtigt werden müssen. Hinzu kommt, daß mit zunehmender Desaktivierung des Katalysators in erhöhtem Umfang Nebenprodukte, wie beispielsweise Cyamelurchlorid, ausgetragen werden und damit einen erhöhten Reinigungsaufwand des Cyanurchlorids erforderlich machen.

Im Hinblick auf die aufgezeigten Probleme war die Fachwelt schon lange daran interessiert, Aktivkohlekatalysatoren mit erhöhter Standzeit aufzufinden und/oder die Betriebsbedingungen so zu variieren, daß die Standzeit erhöht werden kann.

Das US-Patent 3,312,697 lehrt demgemäß ein Verfahren zur Herstellung von Cyanurchlorid unter Einsatz eines Aktivkohlekatalysators mit einer spezifischen Oberfläche von über 1000 m$^2$/g, wobei der Aktivkohlekatalysator durch eine Behandlung mit Säuren und/oder Alkalien und eine nachgeschaltete Wäsche mit Wasser aktiviert wurde. Durch die genannte Behandlung werden anorganische Bestandteile, wie Oxide, Hydroxide und Salze von die Standzeit des Katalysators mindernden Metallen, wie Li, Mg, Ce, Ti, V, Mn, Fe, Ni, Pt, Cu, Zn, Cd, Sn, Pb und Bi, aus der Aktivkohle herausgelöst. Die Standzeit des Katalysators wird in diesem Verfahren ferner dadurch erhöht, daß dem Chlorcyan 0,5 bis 10 Gew.-% Chlor und/oder Phosgen zugesetzt werden.

Im Verfahren gemäß US-Patent 3,707,544 wird die Standzeit dadurch erhöht, daß der Trimerisierungsreaktor mit einem Gemisch aus einer Aktivkohle und einem weniger oder nicht katalytisch wirksamen festen Verdünnungsmittel gemischt ist. Nachteilig an diesem Verfahren ist, daß die Raum-Zeit-Ausbeute gemindert und der Aufwand zur Entsorgung des deaktivierten Katalysators vor allem dann erhöht wird, wenn es sich bei dem Verdünnungsmittel um ein nicht brennbares Material handelt.

Im Verfahren des US-Patents 3,867,382 wird anstelle einer säuregewaschenen Aktivkohle eine aus Kokosnußschalen erzeugte unbehandelte Aktivkohle verwendet. Diese Aktivkohle weist eine innere Oberfläche von 1200 bis 1500 m$^2$/g, ein Mikroporenvolumen von mindestens 0,7 cm$^3$/g und einen Aschegehalt von unter 4 Gew.-% auf. Aufgrund der pflanzlichen Herkunft des Rohstoffs für diese Aktivkohle weist diese einen geringen Schwermetallgehalt auf und macht eine Säurewäsche überflüssig. Dem Dokument läßt sich nicht entnehmen, wie die Mikroporen definiert sind, d. h., ob es sich um alle inneren Poren oder um Mikroporen mit genau definierten Grenzwerten für die Porendurchmesser handelt. Ein erheblicher Nachteil der beispielhaft verwendeten Aktivkohle besteht darin, daß die Schüttdichte und damit die auf das Reaktorvolumen bezogene Einsatzmenge sehr hoch ist und damit die Wirtschaftlichkeit mindert.

E. Wang et al. lehren in J. Beijing Inst. Chem. Technol. 20 (1993) 1, 55-58, daß bei der Auswahl der Katalysatoren für die Chlorcyantrimerisierung verschiedene Parameter berücksichtigt werden müssen, so der Aschegehalt, der Eisengehalt, die spezifische Oberfläche und die Porengrößenverteilung. Die Auswahl einer geeigneten Aktivkohle wird dadurch erschwert, daß sich die Parameter wechselseitig beeinflussen können. Aus diesem Dokument folgt, daß es vorteilhaft ist, eine Kohle zu verwenden, welche eine möglichst hohe spezifische Oberfläche und daher zahlreiche kleine Poren aufweist. Letztere tragen dazu bei, daß die Reaktion an verhältnismäßig zahlreichen aktiven Zentren ablaufen kann. Ausweislich der Abbildungen zur Porengrößenverteilung von zwei unterschiedlichen Aktivkohlen wird nahegelegt, daß die Poren insbesondere einen Durchmesser von kleiner 2 nm aufweisen sollen. Dem Dokument läßt sich jedoch kein Hinweis entnehmen, in welcher Weise die einzelnen Parameter die Standzeit des Katalysators in einer für den kontinuierlichen Betrieb ausgelegten Produktionsanlage beeinflussen.

Aufgabe der vorliegenden Erfindung ist demgemäß, ein verbessertes Verfahren zur Herstellung von Cyanurchlorid durch Trimerisierung von Chlorcyan aufzuzeigen, wobei die Verbesserung in einem verminderten spezifischen Katalysatorverbrauch besteht. Gemäß einer weiteren Aufgabe sollten die Kriterien aufgezeigt werden, anhand derer der Fachmann einen Aktivkohlekatalysator mit verlängerter Standzeit für die gattungsgemäße Reaktion auswählen kann. Weitere Aufgaben lassen sich der nachfolgenden Beschreibung des erfindungsgemäßen Verfahrens entnehmen.

Gefunden wurde ein Verfahren zur Herstellung von Cyanurchlorid, umfassend Trimerisierung von Chlorcyan in Gegenwart einer gewaschenen Aktivkohle mit einer BET-Oberfläche von mindestens 1000 $m^2$/g und einem Fe-Gehalt (berechnet als $Fe_2O_3$) von weniger als 0,15 Gew.-% bei einer Temperatur von mindestens 250 °C, das dadurch gekennzeichnet ist, daß man eine Aktivkohle mit einem effektiven Porenvolumen $V_{eff}$ von gleich oder größer 0,17 ml/g verwendet, wobei $V_{eff}$ aus Poren mit einem Porendurchmesser im Bereich von 0,5 bis 7 nm gebildet werden. Die Unteransprüche richten sich auf bevorzugte Ausführungsformen des Verfahrens.

Es wurde festgestellt, daß die Trimerisierung von Chlorcyan nur in solchen Poren befriedigend abläuft, deren Porendurchmesser im Bereich von 0,5 bis 7 nm, insbesondere 0,5 bis 5 nm liegt; das Porenvolumen dieser Poren soll mindestens 0,17 ml/g betragen. Obgleich die Porenverteilung von Aktivkohlen herstellungsbedingt sehr unterschiedlich sein kann, ist es möglich, das für die Umsetzung erforderliche effektive Porenvolumen $V_{eff}$ aus der Summe eines Volumeninkrements für die Mikroporen mit einem Porendurchmesser von < 2 nm und einem Volumeninkrement der Mesoporen mit einem Porendurchmesser von 2 bis 30 nm zu definieren. Das effektive Porenvolumen kann demgemäß als lineare Funktion dargestellt werden: $V_{eff} = a \cdot V_{mikro} + b \cdot V_{meso}$. Es wurde ferner gefunden, daß die Funktion $V_{eff}$ = 0,25 $\cdot$ 0,50 $V_{mikro}$ + $V_{meso}$ ein geeignetes Auswahlkriterium für eine wirksame Aktivkohle mit einer langen Standzeit ist. Das Mikro- und Mesoporenvolumen wird wie folgt bestimmt:

Das Mikroporenvolumen wird aus der Stickstoff-Adsorptionsisotherme bei der Temperatur des flüssigen Stickstoffs durch Vergleich mit einer Standardisotherme nach dem t-plot-Verfahren von De Boer (vgl. De Boer et al. in J. of Colloid an Interface Science 21, 405-44 (1966)) nach DIN 66135, Teil 2 (Entwurf - April 1998) bestimmt.

Die Bestimmung des Mesoporenvolumens und der Porenverteilung erfolgt aus der Stickstoff-Desorptionsisotherme nach Barett, Joyner und Halenda gemäß DIN 66134 (Februar 1998). Die zur Bestimmung von $V_{mikro}$ und $V_{meso}$ eingesetzte Probe wird vor der Messung 1 h bei 200 °C im Vakuum (kleiner 1,3 Pa) behandelt. Die Messung erfolgt beispielsweise in einem Gerät "ASAP 2400" der Firma Micromeritics, Norcross, Ga. (US). Bei der erfindungsgemäßen Definition des $V_{meso}$ werden nur Mesoporen mit einem Durchmesser von 2 bis 30 nm erfaßt.

Eine besonders hohe Steigerung der Standzeit der Aktivkohle im gattungsgemäßen Verfahren wird dann erzielt, wenn $V_{eff}$ mindestens 0,2 ml/g beträgt. Anhand der Untersuchung zahlreicher unterschiedlicher Aktivkohlen wurde festgestellt, daß ein Maximum des wie oben definierten effektiven Porenvolumens einem Minimum des spezifischen Katalysatorverbrauchs entspricht. Extrem mesoporöse Aktivkohlen als auch extrem mikroporöse Aktivkohlen weisen im mittleren Porenbereich, also im Bereich zwischen 0,5 und 5 nm ein zu geringes Porenvolumen auf, so daß der spezifische Katalysatorverbrauch wesentlich höher ist als bei den erfindungsgemäß zu verwendenden Katalysatoren.

Ein weiteres Merkmal der erfindungsgemäß zu verwendenden Aktivkohlen ist die spezifische Oberfläche (BET-Oberfläche), welche mindestens 1000 $m^2$/g, vorzugsweise mindestens 1200 $m^2$/g, beträgt. Eine hohe Oberfläche ist demgemäß zwar vorteilhaft, sie ist aber kein Kriterium, das einen Rückschluß auf die Katalysatorstandzeit zuläßt. So zeigen unterschiedliche Aktivkohlen mit nahezu gleicher spezifischer Oberfläche sehr große Unterschiede in ihrer Desaktivierungsgeschwindigkeit.

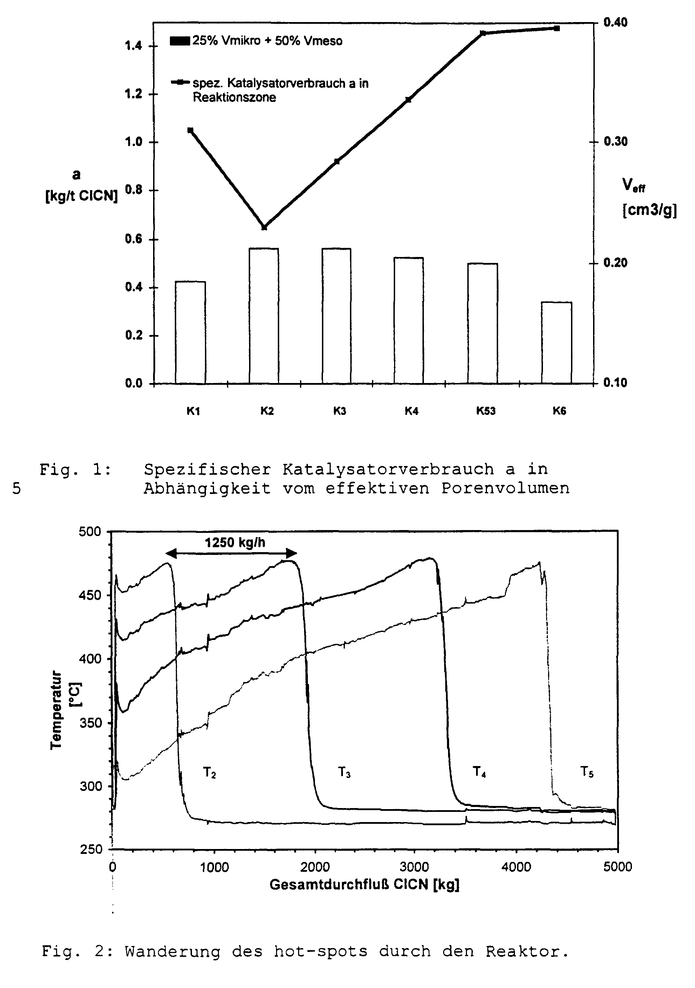

Im Hinblick auf den negativen Einfluß eines hohen Eisengehalts der Aktivkohle sollte dieser, berechnet als $Fe_2O_3$, unter 0,15 vorzugsweise um/unter 0,1 Gew.-% betragen. Obgleich auch ungewaschene Aktivkohlen katalytisch wirksam sind, wird im erfindungsgemäßen Verfahren eine gewaschene, insbesondere eine säuregewaschene Aktivkohle verwendet, weil die Wäsche einerseits eine Möglichkeit darstellt, den Eisen- und übrigen Schwermetallgehalt zu reduzieren und damit die Nebenproduktbildung zu minimieren und andererseits hierdurch das für die Reaktion wichtige Porenvolumen erhöht wird. Im Hinblick auf die Minimierung des spezifischen Katalysatorverbrauchs ist es zudem von Vorteil, eine solche Kohle zu verwenden, deren Schüttdichte gleich oder kleiner 420 g/l beträgt. Bei ausreichender Wirksamkeit des Aktivkohlekatalysators und einem effektiven Porenvolumen von > 0,17 ml/g, vorzugsweise gleich oder > 0,20 ml/g ist es vorteilhaft, wenn die Schüttdichte der Aktivkohle möglichst niedrig ist. Zweckmäßigerweise wird in derartigen Fällen eine Aktivkohle verwendet, deren Schüttdichte gleich oder < 420 g/l, vorzugsweise < 390 g/$cm^3$ ist. Aus der Figur 1, welche die Ergebnisse zahlreicher Untersuchungen zusammenfasst - siehe Beispiele - wird deutlich, in welchem unvorhergesehenen Maße der spezifische Katalysatorverbrauch a (kg Katalysator pro t umgesetztes Chlorcyan) beim Einsatz einer gewaschenen Aktivkohle mit einer BET-Oberfläche von mindestens 1000 $m^2$/g und einem Fe-Gehalt von weniger als 0,15 Gew.-% (berechnet als $Fe_2O_3$) der spezifische Katalysatorverbrauch vom erfindungsgemäß definierten effektiven Porenvolumen abhängt. Der spezifische Katalysatorverbrauch ist insbesondere dann gering, wenn sowohl die Desaktivierungsgeschwindigkeit (die Bestimmungsmethode ist den Beispielen zu entnehmen) als auch gleichzeitig die Schüttdichte des Katalysators möglichst niedrig ist.

**Beispiele**

Die Untersuchungen zur Ermittlung des spezifischen Katalysatorverbrauchs in der Reaktionszone bei der Trimerisierung von Chlorcyan zu Cyanurchlorid wurden in einem Rohrreaktor, der mit dem zu untersuchenden Aktivkohlekatalysator gefüllt war, durchgeführt. Der Rohrreaktor wurde mit einem Wärmeträger gekühlt; die Kühlmitteltemperatur wurde auf 280 °C gehalten. Der Versuchsreaktor war parallel zu einem Betriebsreaktor geschaltet. Das entstehende gasförmige Cyanurchlorid wurde nach Verlassen des Reaktors kondensiert und das flüssige Produkt zur Überführung in den festen Aggregatzustand in gekühlten Kammern versprüht.

Das Verhältnis der Reaktorlänge zum Reaktorquerschnitt betrug 39. Im kontinuierlichen Betrieb bildete sich ein Temperaturprofil entlang der Längsachse des Reaktors aus. Dieses Profil umfasst eine Erwärmungszone, eine Reaktionszone und eine Abkühlungszone. Das Maximum der Reaktionszone, dessen Temperatur mit wachsendem Durchsatz ansteigt, wandert mit wachsender Katalysatordesaktivierung in Strömungsrichtung voran. Die Desaktivierungsgeschwindigkeit ($u_{Desakt}$) wurde bestimmt, indem aus entlang des Reaktors angeordneten Temperaturmeßstellen zeitabhängige Temperaturprofile erstellt wurden.

Die Figur 2 zeigt, daß mit zunehmender Betriebsdauer der hot-spot der Reaktionszone durch das Raster der hintereinander angeordneten Meßstellen wandert. Die eigentliche Bestimmung der Desaktivierungsgeschwindigkeit wurde nach einer sogenannten Katalysatorvordesaktivierung begonnen - zu diesem Zeitpunkt baute sich in der Nähe des Reaktoreingangs der "hot-spot" auf. Die Katalysatorvordesaktivierung dauerte bei einem Durchsatz von 1,1 kg Chlorcyan pro Stunde etwa 12 Stunden. Figur 2 zeigt einen typischen Verlauf der Desaktivierung. Aus dem Abstand der Temperaturmeßstellen und der mittleren Chlorcyanmenge (gemessen von Maximum zu Maximum) läßt sich die Desaktivierungsgeschwindigkeit in cm/t ClCN bestimmen. Der spezifische Katalysatorverbrauch in der Reaktionszone läßt sich aus der Desaktivierungsgeschwindigkeit ($v_{desakt.}$), der Reaktorgeometrie (Querschnittsfläche F) sowie der Schüttdichte ρ nach folgender Gleichung ermitteln:

$$a \left[ \frac{kg\ Kat}{t\ ClCN} \right] = u_{Desakt} \left[ \frac{cm}{t\ ClCN} \right] \cdot F\ [cm^2] \cdot \rho \left[ \frac{kg}{m^3} \right]$$

Eingesetzt wurden die in der Tabelle 1 charakterisierten Aktivkohlen.

EP 1 183 244 B1

Tabelle 2 zeigt die Desaktivierungsgeschwindigkeit u und den spezifischen Katalysatorverbrauch a in der Reaktionszone unter Verwendung der in Tabelle 1 angegebenen Aktivkohlen, wobei in allen Versuchen der ClCN-Durch-

Tabelle 1:   Eingesetzte Aktivkohlekatalysatoren

| Katalysator (Nr.) | Rohstoff | Wäsche | Asche-gehalt (Gew.-%) | Fe-Gehalt (als $Fe_2O_3$) (Gew.-%) | Schütt-dichte (g/l) | BET ($m^2/g$) | Porenvolumen ($cm^3/g$) | | |
|---|---|---|---|---|---|---|---|---|---|
| | | | | | | | $V_{mikro}$ | $V_{meso}$ | $V_{eff}$ *) |
| K1 | Torf | + | 1,67 | 0,00 | 403 | 1016 | 0,38 | 0,18 | 0,185 |
| K2 | Torf | + | 2,45 | 0,07 | 346 | 1453 | 0,63 | 0,11 | 0,213 |
| K3 | Steinkohle | + | 2,24 | 0,03 | 410 | 1217 | 0,51 | 0,17 | 0,212 |
| K4 | Holz | + | 2,18 | 0,28 | 375 | 1523 | 0,64 | 0,09 | 0,205 |
| K5 | Kiefer | – | 8,01 | 0,16 | 406 | 1290 | 0,58 | 0,11 | 0,200 |
| K6 | Kokosnuß | + | 0,42 | 0,00 | 373 | 1459 | 0,59 | 0,04 | 0,157 |
| K7 | Torf | + | 2,46 | 0,07 | 434 | 1213 | 0,50 | 0,08 | 0,165 |
| K8 | Kokosnuß | + | 1,66 | 0,01 | 430 | 1110 | 0,45 | 0,07 | 0,147 |

*) $V_{eff} = 0,25\ V_{mikro} + 0,5\ V_{meso}$

satz 4,4 kg pro Stunde betrug.

Tabelle 2:

| Desaktivierungsgeschwindigkeit V und spezifischer Katalysatorverbrauch a in der Reaktionszone | | |
|---|---|---|
| Katalysator Nr. | u (cm/t ClCN) | a kg Kat./t ClCN |
| K1 | 29 | 1,05 |
| K2 | 21 | 0,65 |
| K3 | 25 | 0,92 |
| K4 *) | 35 | 1,18 |
| K5 *) | 40 | 1,46 |
| K6 *) | 35 | 1,18 |
| K7 *) | 28 | 1,09 |

Temperatur des Wärmeträgers 280 °C
*) nicht erfindungsgemäßer Aktivkohlekatalysator

Die Versuche zeigen, daß der spezifische Katalysatorverbrauch in der Reaktionszone maßgeblich vom effektiven Porenvolumen und der Schüttdichte des Katalysators abhängt. Durch einen verminderten Katalysatorverbrauch werden nicht nur die Kosten für den Katalysator vermindert, sondern gleichzeitig wird die Verfügbarkeit der Anlage durch verminderte Stillstandszeiten erhöht, wodurch die Wirtschaftlichkeit des Verfahrens gleichfalls ansteigt.

## Patentansprüche

1.　Verfahren zur Herstellung von Cyanurchlorid, umfassend Trimerisierung von Chlorcyan in Gegenwart einer gewaschenen Aktivkohle mit einer BET-Oberfläche von mindestens 1000 $m^2$/g und einem Fe-Gehalt von weniger als 0,15 Gew.-% (berechnet als $Fe_2O_3$) bei einer Temperatur von mindestens 250 °C,
**dadurch gekennzeichnet,**
**daß** man eine Aktivkohle mit einem effektiven Porenvolumen $V_{eff}$ von gleich oder größer 0,17 ml/g verwendet, wobei $V_{eff}$ aus Poren mit einem Porendurchmesser im Bereich von 0,5 bis 7 nm gebildet werden.

2.　Verfahren nach Anspruch 1,
**dadurch gekennzeichnet,**
**daß** man eine Aktivkohle verwendet, deren effektives Porenvolumen $V_{eff}$ gebildet ist aus der Summe $V_{eff}$ = 0,25 . $V_{mikro}$ + 0,5 $V_{meso}$, wobei $V_{mikro}$ Poren mit einem Durchmesser von kleiner 2 nm und $V_{meso}$ Poren mit einem Durchmesser von 2 bis 30 nm umfaßt.

3.　Verfahren nach Anspruch 1 oder 2,
**dadurch gekennzeichnet,**
**daß** $V_{eff}$ der verwendeten Aktivkohle mindestens 0,2 ml/g beträgt.

4.　Verfahren nach einem der Ansprüche 1 bis 3,
**dadurch gekennzeichnet,**
**daß** die zu verwendende Aktivkohle eine Schüttdichte von gleich oder kleiner 420 g/l aufweist.

5.　Verfahren nach einem der Ansprüche 1 bis 4,
**dadurch gekennzeichnet,**
**daß** die zu verwendende Aktivkohle eine BET-Oberfläche von mindestens 1200 $m^2$/g aufweist und $V_{eff}$ mindestens 0,2 ml/g beträgt.

## Claims

1.　Process for producing cyanuric chloride, comprising trimerisation of cyanogen chloride in the presence of a washed activated carbon having a BET surface area of at least 1000 $m^2/g$ and an Fe content of less than 0.15 wt.%, (calculated as $Fe_2O_3$) at a temperature of at least 250 °C,
**characterised in that**
an activated carbon having an effective pore volume $V_{eff}$ of equal to or greater than 0.17 ml/g is used, $V_{eff}$ being obtained from pores having a pore diameter in the range of 0.5 to 7 nm.

2. Process according to claim 1,
**characterised in that**
an activated carbon is used, whose effective pore volume $V_{eff}$ is formed from the sum $V_{eff} = 0.25 \cdot V_{micro} + 0.5 V_{meso}$, $V_{micro}$ comprising pores having a diameter of less than 2 nm and $V_{meso}$ comprising pores having a diameter of 2 to 30 nm.

3. Process according to claim 1 or 2,
**characterised in that**
$V_{eff}$ of the activated carbon used is at least 0.2 ml/g.

4. Process according to one of claims 1 to 3,
**characterised in that**
the activated carbon to be used has a bulk density of equal to or less than 420 g/l.

5. Process according to one of claims 1 to 4,
**characterised in that**
the activated carbon to be used has a BET surface area of at least 1200 $m^2/g$ and $V_{eff}$ is at least 0.2 ml/g.

**Revendications**

1. Procédé pour la préparation de chlorure cyanurique, comprenant la trimérisation de chlorure de cyanogène en présence de charbon actif lavé présentant une surface BET d'au moins 1000 $m^2/g$ et une teneur en Fe inférieure à 0,15% en poids (calculé sous forme de $Fe_2O_3$) à une température d'au moins 250°C, **caractérisé en ce qu'**on utilise du charbon actif présentant un volume effectif de pores $V_{eff}$ égal ou supérieur à 0,17 ml/g, $V_{eff}$ étant formé par des pores présentant un diamètre de pores dans la plage de 0,5 à 7 nm.

2. Procédé selon la revendication 1, **caractérisé en ce qu'**on utilise du charbon actif dont le volume effectif des pores $V_{eff}$ est formé par la somme $V_{eff} = 0,25.V_{micro} + 0,5.V_{méso}$, $V_{micro}$ comprenant les pores présentant un diamètre inférieur à 2 nm et $V_{méso}$ comprenant les pores présentant un diamètre de 2 à 30 nm.

3. Procédé selon la revendication 1 ou 2, **caractérisé en ce que** $V_{eff}$ du charbon actif utilisé est d'au moins 0,2 ml/g.

4. Procédé selon l'une quelconque des revendications 1 à 3, **caractérisé en ce que** le charbon actif à utiliser présente une densité apparente égale ou inférieure à 420 g/l.

5. Procédé selon l'une quelconque des revendications 1 à 4, **caractérisé en ce que** le charbon actif à utiliser présente une surface BET d'au moins 1200 $m^2/g$ et que $V_{eff}$ est d'au moins 0,2 ml/g.

Fig. 1: Spezifischer Katalysatorverbrauch a in
Abhängigkeit vom effektiven Porenvolumen

Fig. 2: Wanderung des hot-spots durch den Reaktor.